# EUROPÄISCHE PATENTSCHRIFT

(11) **EP 0 853 935 B1**
(45) Veröffentlichungstag und Bekanntmachung des Hinweises auf die Patenterteilung: **26.09.2001**
(21) Anmeldenummer: 97122619.6
(22) Anmeldetag: 22.12.1997
(51) Int. Cl.: A61F 13/58, A61F 13/56

(54) **Windelverschluss mit einem Fasteningträger**
Fastener for disposable diaper
Attache pour couche à jeter

(30) Priorität: 16.01.1997 DE 19701241
(43) Veröffentlichungstag der Anmeldung: 22.07.1998
(73) Patentinhaber: Koester GmbH & Co. KG, 96146 Altendorf (DE)
(72) Erfinder: Bräunig, Werner, 97514 Oberaurach (DE); Neugebauer, Robert, 91077 Neunkirchen a. Brand (DE); Colakovic, Dusko, 91056 Erlangen (DE)
(74) Vertreter: Castell, Klaus, Dr.

(56) Entgegenhaltungen:
- EP-A- 0 832 631
- DE-A- 3 419 621
- US-A- 4 111 205
- US-A- 4 643 729
- US-A- 4 834 820

## Beschreibung

Die Erfindung betrifft einen Windelverschluß mit einem Fasteningträger, der einen Befestigungsbereich zur Befestigung des Fasteningträgers an einer Windel, einen Fasteningmittel umfassenden Fasteningbereich und einen zwischen dem Befestigungsbereich und dem Fasteningbereich angeordneten Zwischenbereich umfaßt.

Derartige Windelverschlüsse dienen dem Verschließen von Windeln zum einen beim Gebrauch am Körper und zum anderen nach deren Gebrauch zur Entsorgung. Hierbei handelt es sich um tapeartige Fasteningträger, die in einem Befestigungsbereich an der Windel befestigt sind und die an ihrem anderen Ende ein Fasteningmittel, wie zum Beispiel eine Klebezone oder den männlichen Teil eines Klettverschlußsystemes umfassen. Letztgenannte mechanische Windelverschlußsysteme benötigen einen weiblichen Teil, der an entsprechenden Stellen der Windel vorgesehen ist.

US-A-4,643,729 zeigt einen Windelverchluß mit einem dehnlosren mittleren Teil und zwei feste laterale Teile. Die zwei lateralen Teile sind mit einem Unterstützungsband verbunden aber mit einer Sollbruchstelle versehen, die zum Gebrauch aufgerissen wird.

Bei neuen Windelgenerationen wird die gesamte Windelaußenhaut mit textilem Material versehen, um die Windel angenehmer und weicher darzustellen. Wird auf solchen Windeln (mit clothlike backsheet) mit mechanischen Verschlußsystemen gearbeitet, kann das Zusammenrollen und Verschließen der Windeln nach deren Gebrauch auch ohne zusätzliche, weibliche Bestandteile oder aber zusätzliche, an dem Windelverschluß befindliche und auf einer glatten Windelaußenhaut anhaftende Klebezonen erfolgen, wenn sich die männlichen Bestandteile des Klettverschlußsystemes ausreichend auf dieser Windelaußenhaut fixieren lassen.

Es ist Aufgabe vorliegender Erfindung, einen Windelverschluß bereit zu stellen, welcher sich problemlos herstellen und verarbeiten läßt.

Als Lösung schlägt die Erfindung einen gattungsgemäßen Windelverschluß vor, der eine nicht wieder fixierbare Halteeinrichtung zur Fixierung des Fasteningbereiches umfaßt.

Durch eine derartige Anordnung wird ein Windelverschluß bereit gestellt, das eine ausreichende Fixierung des Fasteningbereichs an der Windel vor Gebrauch derselben erreicht, so daß der Fasteningträger nach dem Applizieren der Windel nicht ungewollt aufspringt. Wird als Fasteningmittel ein Teil eines Klettverschlusses, zum Beispiel ein Haken- oder Pilzkopfmaterial verwendet, so wird durch die nicht wieder fixierbare Halteeinrichtung eine ausreichende Fixierung des Fasteningbereichs und somit auch des Fasteningträgers gewährleistet, auch wenn die Fasteningmittel selbst eine ausreichende Fixierung, zum Beispiel während der maschinellen Weiterverarbeitung einer mit einem erfindungsgemäßen Windelverschluß versehenen Windeln nicht gewährleisten. Insbesondere ist es auch möglich, den Windelverschluß derart auszugestalten, daß vor Gebrauch der Windeln die Fasteningmittel selbst nicht an der Windel fixiert sind. Es kann also auf ein entsprechendes Gegenstück, wie zum Beispiel eine Klebefläche oder den weiblichen Teil eines Klettverschlusses in diesem Windelbereich verzichtet werden.

Darüberhinaus kann, wenn ein mechanisches Windelverschlußsystem, wie zum Beispiel ein Klettverschlußsystem bei einer textilen Windelaußenhaut (clothlike backsheet) Verwendung findet, auf zusätzliche Klebezonen verzichtet werden, da diese zum einen zum Verschließen der Windel beim Gebrauch oder nach deren Gebrauch nicht notwendig sind und da zum anderen durch die nicht wieder fixierbare Halteeinrichtung eine ausreichende Fixierung des Fasteningbereiches und somit des Fasteningträgers an der Windel gewährleistet ist.

Eine besonders einfache Realisierung eines erfindungsgemäßen Windelverschlusses folgt, wenn dieser ein Haltetape umfaßt, welches an der Windel befestigt und über die Halteeinrichtung mit dem Fasteningbereich des Fasteningträgers verbunden ist. Haltetape, Fasteningträger und die diese beiden verbindende Halteeinrichtung können gemeinsam hergestellt und anschließend in einem Arbeitsgang an einer Windel appliziert werden. Auf diese Weise wird eine einfache und kostengünstige Weiterverarbeitung des erfindungsgemäßen Windelverschlusses gewährleistet.

Vorteilhafter Weise kann das Haltetape auf Höhe des Zwischenbereichs an der Windel befestigt und die Halteeinrichtung an der dem Zwischenbereich zugewandten Seite des Fasteningbereichs angeordnet sein. Dies ermöglicht eine besonders kompakte Ausgestaltung des erfindungsgemäßen Windelverschlusses, da sich dessen räumliche Ausdehnung auf die notwendige, räumliche Ausdehnung des Fasteningträgers beschränkt, während das Haltetape und somit auch die Halteeinrichtung zwischen Fasteningträger und Windel angeordnet sein können und somit keinen zusätzlichen Raum beanspruchen.

Erstreckt sich das Haltetape bis in den Fasteningbereich und ist an diesem befestigt und wird die Halteeinrichtung durch die strukturelle Einheit des Haltetapes gebildet und durch Zerstören der strukturellen Einheit gelöst, folgt zum einen eine einfache und somit kostengünstige Realisierung der erfindungsgemäßen Halteeinrichtung, die zudem die Gefahr einer Faltenbildung zwischen Haltetape und Fasteningträger reduziert und ein unerwünschtes Aufspringen des Fasteningträgers während der Herstellung des erfindungsgemäßen Windelverschlusses, zum Beispiel während des Schneidens, und auch während oder nach dem Applizieren desselben an einer Windel erfolgreich verhindert.

Um ein Loslösen der erfindungsgemäßen, nicht wieder fixierbaren Halteeinrichtung zu erleichtern, kann das Haltetape derart an der Windel und dem Fasteningträger befestigt sein, daß es in unmittelbarer Umgebung der Halteeinrichtung weder an der Windel noch an den Fasteningträger befestigt ist. Hierdurch können die Halteeinrichtung leicht von dem Fasteningträger und der Windel entfernt und die zum Lösen der Halteeinrichtung notwendigen Kräfte einfacher aufgebracht werden.

Eine gute Fixierung des Haltetapes und somit auch des Fasteningbereichs an der Windel wird gewährleistet, wenn sich das Haltetape zumindest über die Länge des Zwischenbereichs erstreckt und wenigstens über diese Länge an der Windel befestigt ist. Es versteht sich, daß hierbei in den Rand- bzw. Übergangsbereichen zwischen Haltetape bzw. Zwischenbereich und Fasteningbereich auf eine Befestigung verzichtet werden kann, zum Beispiel um die unmittelbare Umgebung der Halteeinrichtung freizugeben, ohne daß hierdurch die Fixierung wesentlich beeinträchtigt wird.

Erstreckt sich das Haltetape bis in den Befestigungsbereich des Fasteningträgers und ist es in diesem Bereich an der Windel einerseits und an dem Fasteningträger andererseits befestigt, kann die Gefahr einer Faltenbildung zwischen Fasteningträger und Haltetape während der Herstellung des Windelverschlusses als auch bei dessen Applizieren an einer Windel reduziert werden. Insbesondere wenn sich das Haltetape sowohl bis in den Fasteningbereich als auch bis in den Befestigungsbereich des Fasteningträgers erstreckt, wird eine derartige Gefahr vermindert. In diesem Falle ist auch die Gefahr eines unerwünschten Aufspringens des Fasteningträgers beim Herstellen oder Verarbeiten des Windelschlusses, zum Beispiel bei dessen Schneiden, und auch beim Applizieren desselben an einer Windel in vorteilhafter Weise vermindert.

Eine einfache Ausgestaltung der erfindungsgemäßen Halteeinrichtung folgt, wenn diese als Perforation, zum Beispiel des Haltetapes gebildet ist. Eine derartige Perforation kann in einfacher Weise den Erfordernissen einer ausreichenden Fixierung des Fasteningbereichs durch Wahl ihrer Geometrie angepaßt werden. So kann diese linear, wellenförmig, zick-zack-förmig oder punktuell ausgestaltet sein. Auch die Größenverhältnisse der Löcher zu den Zwischenräumen ermöglichen eine Einstellung des Verhältnisses zwischen Haltekraft und den zum Lösen der Halteeinrichtung, also zum Zerreißen der Perforation, notwendigen Kräften.

Eine kompakte Ausgestaltung des erfindungsgemäßen Windelverschlusses, die eine minimale Gefahr von Faltenbildung oder Aufspringen bei der Herstellung oder Weiterverarbeitung des Windelverschlusses gewährleistet, kann dadurch erreicht werden, daß die Perforation an der dem Zwischenbereich zugewandten Seite des Fasteningbereichs vorgesehen ist.

In konkreter Ausgestaltung kann das Haltetape in dem Befestigungsbereich und in dem Fasteningbereich an dem Fasteningträger befestigt sein, wobei die Fasteningmittel an der dem Fasteningträger abgewandten Seite des Haltetapes befestigt sind und das Haltetape an der dem Fasteningträger abgewandten Seite auf Höhe des Befestigungsbereichs und des Zwischenbereichs mit Befestigungsmitteln versehen ist. Hierdurch wird die Herstellung und Weiterverarbeitung des erfindungsgemäßen Windelverschlusses vereinfacht. Dieser kann in kompakter Weise seperat hergestellt werden und braucht dann nur mit den Befestigungsmitteln an die Windel appliziert werden.

Als Befestigungsmittel können alle bekannten Befestigungsmittelarten, insbesondere Kleben und Verschweißen, dienen. Dieses gilt zum einen für die Befestigung des Fasteningträgers, und zwar an der Windel oder auch dem Haltetape und zum anderen für die Befestigung des Haltetapes an der Windel bzw. dem Fasteningträger.

Es versteht sich, daß als Fasteningträger bzw. auch als Haltetape alle bekannten Trägermaterialien für Windelverschlüsse, wie zum Beispiel Folien, Vliese oder Papiere, Anwendung finden können. Auch versteht es sich, daß die konkrete Wahl der Fasteningmittel, zum Beispiel der männliche Teil eines Klettverschlusses, die Erfindung als solche nicht berühren, so daß auch andere Fasteningmittel Anwendung finden können.

Der Windelverschluß kann desweiteren mit einer Handhabe versehen sein, die ein Öffnen und Schließen desselben erleichtert. Vorteilhafterweise ist die Handhabe derart ausgestaltet, daß sie auch ein Lösen der Halteeinrichtung vereinfacht. Insbesondere kann die Handhabe auf der der Halteeinrichtung abgewandten Seite des Fasteningbereichs angeordnet sein, so daß zum Lösen der Halteeinrichtung zusätzlich der Fasteningbereich als Handhabe genutzt werden kann.

Weitere Vorteile, Eigenschaften und Ziele vorliegender Erfindung folgen aus nachfolgender Beschreibung und anliegender Zeichnung, in welcher beispielhaft eine Ausführungsform eines erfindungsgemäßen Windelverschlusses dargestellt ist. In der Zeichnung zeigen,
- Figur 1: einen erfindungsgemäßen Windelverschluß im Schnitt,
- Figur 2: eine aufgeschnittene Materialbahn zur Herstellung von Windelverschlüssen nach Figur 1,
- Figur 3: den an eine Windel applizierten Windelverschluß nach Figur 1 bei fixierter Halteeinrichtung im Schnitt und
- Figur 4: die Anordnung nach Figur 3 bei gelöster Halteeinrichtung.

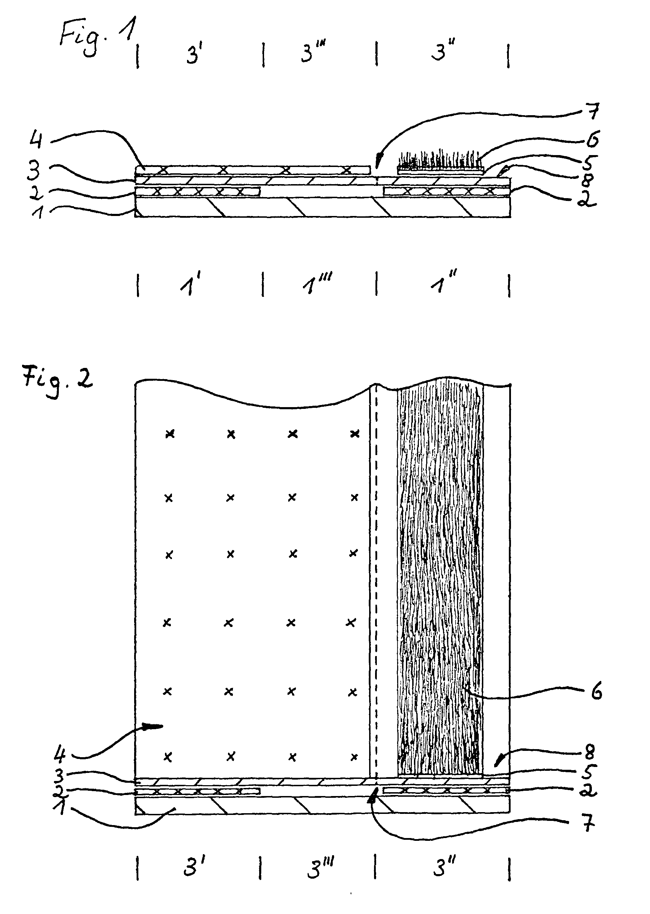

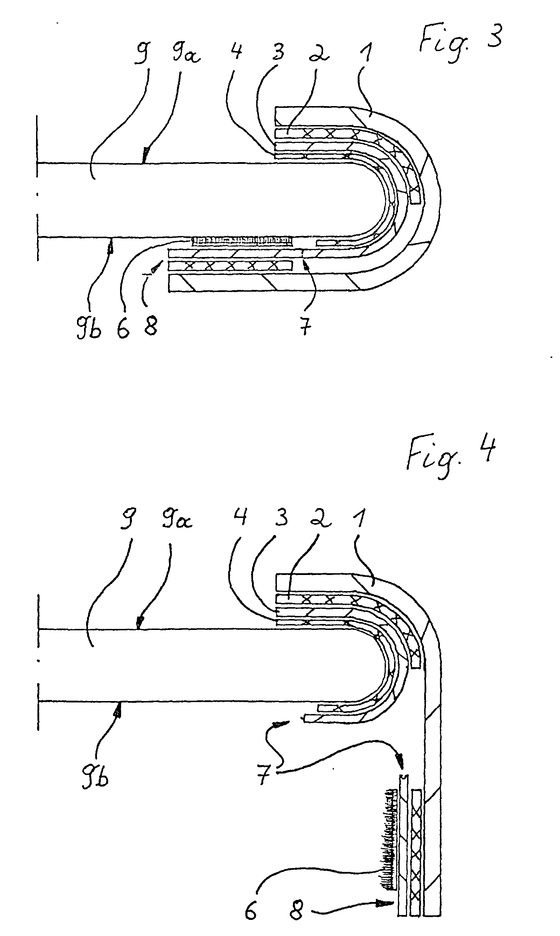

Der beispielhaft dargestellte Windelverschluß umfaßt einen Fasteningträger 1, an welchem mittels Verschweißungen 2 ein Haltetape 3 befestigt ist. Dieses weist eine Klebstoffbeschichtung 4 zur Befestigung einer Windel 9 (siehe Figur 3 und 4) an seiner dem Fasteningträger 1 abgewandten Seite auf.

Mittels einer Verschweißung 5 ist das Haltetape 3 an seiner dem Fasteningträger 1 abgewandten Seite mit Hakenmaterial 6 als Fasteningmittel versehen.

Zwischen der Klebstoffbeschichtung 4 und dem Hakenmaterial 6 weist das Haltetape 3 eine Perforation 7 auf. In unmittelbarer Umgebung der Perforation 7 ist das Haltetape 3 weder mit einer Klebstoffbeschichtung 4 versehen, noch mit dem Fasteningträger 1 verschweißt. Auch zu dem Hakenmaterial 6 ist von der Perforation 7 ein Abstand belassen.

Ebenso findet sich auf der anderen Seite des Hakenmaterials 6 eine freie Oberfläche des Haltetapes 3, die als Handhabe 8 zum Öffnen und Schließen des Windelverschlusses sowie zum Lösen der Halteeinrichtung, also der Perforation 7, dient.

Der Windelverschluß umfaßt einen Befestigungsbereich 1', in welchem der Fasteningträger 1 an dem Haltetape 3 und somit an der Windel 9 befestigt ist. Desweiteren weist der Windelverschluß einen Fasteningträger 1" auf, in welchem das Fasteningmittel, nämlich das Hakenmaterial 6, angebracht ist. Zwischen dem Befestigungsbereich 1' und dem Fasteningbereich 1" befindet sich ein Zwischenbereich 1''', der eine Beweglichkeit zwischen Befestigungsbereich 1' und Fasteningbereich 1" ermöglicht und derart bemessen ist, daß die Windel 9 sicher verschlossen werden kann. Entsprechend den vorgenannten Bereichen weist das Haltetape 3 einen hinteren Haltetapebereich 3', einen Fasteningbereich 3" und einen vorderen Haltetapebereich 3''' auf. Der hintere Haltetapebereich 3' ist mit dem Fasteningträger 1 verschweißt und gewährleistet auf diese Weise eine Befestigung des Windelverschlusses an der Windel 9. Der Fasteningbereich 3" ist ebenfalls mit dem Fasteningträger 1 verschweißt und trägt das Hakenmaterial 6. Nach dem Lösen der Perforation 7 verbleibt der Fasteningbereich 3" an dem Fasteningträger 1, so daß dieser in bestimmungsgemäßer Weise zum Verschließen der Windel bewegt werden kann. Der vordere Haltetapebereich 3''' ist nicht mit dem Fasteningträger 1 verbunden und ermöglicht auf diese Weise eine freie Beweglichkeit des Fasteningträgers 1 im Zwischenbereich 1'''.

Der Windelverschluß kann durch Trennen von Bahnen, wie in Figur 2 dargestellt, hergestellt werden. Durch die beidseitige Verbindung des Haltetapes 3 mit dem Fasteningträger 1 über die Verschweißungen 2 wird die Gefahr einer Faltenbildung während des Herstellungsprozesses, des Schneidens oder des Applizierens an der Windel auf ein Minimum reduziert. Darüberhinaus verhindert die Perforation 7 zwischen den hinteren und vorderen Haltetapebereichen 3' und 3''' sowie dem Fasteningbereich 3" ein Aufspringen des Fasteningträgers 1 beim Schneiden des Windelverschlusses oder dessen Applizieren an eine Windel 9.

Der Windelverschluß kann zum Beispiel in der in Figuren 3 und 4 dargestellten Weise an eine Windel 9 appliziert werden. Hierbei umgreift der Windelverschluß in seinem Befestigungsbereich 1' und seinem Zwischenbereich 1''' einen Rand der Windel 9 von der Windelaußenseite 9a ausgehend bis hin zur Windelinnenseite 9b. Durch die Klebstoffbeschichtung 4 wird eine feste Verbindung der vorderen und hinteren Haltetapebereiche 3' und 3"' an der Windel gewährleistet. Das durchgehende Haltetape 3 bedingt eine ausreichende Fixierung des Haltetapes 3 an der Windelinnenseite 9b. Durch Anhebung der Handhabe 8 kann der Fasteningbereich 1" aufgenommen und die Perforation 7 leicht durchtrennt werden. Auf diese Weise steht der erfindungsgemäße Windelverschluß in gewohnter Weise zur Verfügung, während die vorderen und hinteren Haltetapebereiche 3' und 3''' auf der Windeloberfläche und der Fasteningbereich 3" des Haltetapes 3 im Fasteningbereich 1'' zwischen Hakenmaterial 6 und Fasteningträger 1 verbleiben.

Es versteht sich, daß auch eine andere Handhabe als die Handhabe 8 des vorliegenden Ausführungsbeispiels Verwendung finden kann. Vorliegende Handhabe 8 zeichnet sich durch eine besonders einfache Herstellung aus, da sie lediglich aus einem freien, windelseitigen Oberflächenbereich des Haltetapes 3 besteht. Die vorliegende Anordnung ermöglicht es desweiteren in vorteilhafter Weise, daß der Nutzer auch das Hakenmaterial 6 selbst zur Handhabung des Windelverschlusses nutzt.

## Patentansprüche

1. Windelverschluss mit einem Fasteningträger (1), der einen Befestigungsbereich (1') zur Befestigung des Fasteningträgers (1) an einer Windel (9), einen Fasteningmittel (6) umfassenden Fasteningbereich (1'') und einen zwischen dem Befestigungsbereich (1') und dem Fasteningbereich (1'') angeordneten Zwischenbereich (1''') umfasst, ***gekennzeichnet durch*** eine nicht wieder fixierbare Halteeinrichtung (7) zur Fixierung des Fasteningbereiches (1''), wobei ein Haltetape (3) an der Windel (9) auf Höhe des Zwischenbereichs (1''') an der Windel (9) befestigt und über die Halteeinrichtung (7) an der dem Zwischenbereich (1''') zugewandten Seite des Fasteningbereichs (1'') des Fasteningträgers (1) mit dem Fasteningbereich (1'') des Fasteningträgers (1) verbunden ist.

2. Windelverschluss nach Anspruch 1, ***dadurch gekennzeichnet, dass*** das Haltetape (3) in dem Befestigungsbereich (1') und in dem Fasteningbereich (1'') an dem Fasteningträger (1) befestigt ist, wobei die Fasteningmittel (6) an der dem Fasteningträger (1) abgewandten Seite des Haltetapes (3) befestigt sind und das Haltetape (3) an der dem Fasteningträger (1) abgewandten Seite auf Höhe des Befestigungsbereichs (1') und des Zwischenbereichs (1''') mit Befestigungsmittel versehen ist.

3. Windelverschluss nach Anspruch 1 oder 2, ***dadurch gekennzeichnet, dass*** sich das Haltetape (3) bis in den Fasteningbereich (1") erstreckt und an diesem befestigt ist, wobei die Halteeinrichtung (7) durch die strukturelle Einheit des Haltetapes (3) gebildet und durch Zerstören der strukturellen Einheit gelöst wird.

4. Windelverschluss nach einem der vorhergehenden Ansprüche, ***dadurch gekennzeichnet, dass*** sich das Haltetape (3) zumindest über die Länge des Zwischenbereichs (1''') erstreckt und wenigstens über diese Länge an der Windel (9) befestigt ist.

5. Windelverschluss nach einem der vorhergehenden Ansprüche, ***dadurch gekennzeichnet, dass*** sich das Haltetape (3) bis in den Befestigungsbereich (1') des Fasteningträgers (1) erstreckt und in diesem Bereich (1') an der Windel (9) einerseits und an dem Fasteningträger (1) andererseits befestigt ist.

6. Windelverschluss nach einem der vorhergehenden Ansprüche, ***dadurch gekennzeichnet, dass*** die Halteeinrichtung (7) eine Perforation, vorzugsweise des Haltetapes (3), umfasst.

7. Windelverschluss nach Anspruch 6, ***dadurch gekennzeichnet, dass*** die Perforation an der dem Zwischenbereich (1''') zugewandten Seite des Fasteningbereiches (1'') vorgesehen ist.

## Claims

1. Diaper (nappy) closure device with a fastening carrier (1), comprising a securing area (1') for securing the fastening carrier (1) to a diaper (nappy) (9), a fastening area (1") comprising fastening means (6), and an intermediate area (1''') arranged between the securing area (1') and the fastening area (1"), **characterised by** a retaining device (7), not capable of being again fixed in position, for the purpose of fixing the fastening area (1"), whereby a retaining tape (3) is secured to the diaper (nappy) (9) at the height of the intermediate area (1''') at the diaper (nappy), and which is connected, via the retaining device (7) on the side of the fastening area (1") of the fastening carrier (1) turned towards the intermediate area (1'''), to the fastening area (1") of the fastening carrier (1).

2. Diaper (nappy) closure device according to Claim 1, **characterised in that** the retaining tape (3) is secured in the securing area (1') and in the fastening area (1") to the fastening carrier (1), whereby the fastening means (6) are secured to the side of the retaining tape (3) turned away from the fastening carrier (1), and the retaining tape (3) is provided with securing means on the side turned away from the fastening carrier (1), at the height of the securing area (1') and the intermediate area (1''').

3. Diaper (nappy) closure device according to Claim 1 or 2, **characterised in that** the retaining tape (3) extends into the fastening area (1") and is secured to this, whereby the retaining device (7) is formed by the structural unit of the retaining tape (3) and is released by the destruction of the structural unit.

4. Diaper (nappy) closure device according to one of the foregoing claims, **characterised in that** the retaining tape (3) extends at least over the length of the intermediate area (1''') and is secured to the diaper (nappy) (9) at least over this length.

5. Diaper (nappy) closure device according to one of the foregoing claims, **characterised in that** the retaining tape (3) extends into the securing area (1') of the fastening carrier (1) and in this area (1') is secured to the diaper (nappy) on the one side and to the fastening carrier (1) on the other side.

6. Diaper (nappy) closure device according to one of the foregoing claims, **characterised in that** the retaining device (7) comprises a perforation, for preference of the retaining tape (3).

7. Diaper (nappy) closure device according to Claim 6, **characterised in that** the perforation is provided on the side of the fastening area (1") turned towards the intermediate area (1''').

## Revendications

1. Fermeture de couche ayant un support d'attache (1), qui comprend une zone de fixation (1') pour fixer le support d'attache (1) à une couche (9), une zone d'attache (1") comprenant des moyens d'attache (6) et une zone intermédiaire (1''') disposée entre la zone de fixation (1') et la zone d'attache (1"), **caractérisée par** un dispositif de retenue (7) ne pouvant être refixé, pour fixer la zone d'attache (1"), une bande de retenue (3) sur la couche (9) étant fixée à la hauteur de la zone intermédiaire (1''') sur la couche (9) et étant liée à la zone d'attache (1") du support d'attache (1) par le dispositif de retenue (7) sur la face tournée vers la zone intermédiaire (1'''), de la zone d'attache (1") du support d'attache (1).

2. Fermeture de couche selon la revendication 1, **caractérisée en ce que** la bande de retenue (3) est fixée dans la zone de fixation (1') et dans la zone d'attache (1") sur le support d'attache (1), les moyens d'attache (6) étant fixés sur la face détournée du support d'attache (1), de la bande de retenue (3) et la bande de retenue (3) étant munie de moyens de fixation sur la face détournée du support d'attache (1), à la hauteur de la zone de fixation (1') et de la zone intermédiaire (1''').

3. Fermeture de couche selon la revendication 1 ou 2, **caractérisée en ce que** la bande de retenue (3) s'étend jusque dans la zone d'attache (1") et est fixée sur celle-ci, le dispositif de retenue (7) étant formé par l'unité structurale de la bande de retenue (3) et se détachant par la destruction de l'unité structurale.

4. Fermeture de couche selon l'une quelconque des revendications précédentes, **caractérisée en ce que** la bande de retenue (3) s'étend au moins sur la longueur de la zone intermédiaire (1''') et est fixée au moins sur cette longueur sur la couche (9).

5. Fermeture de couche selon l'une quelconque des revendications précédentes, **caractérisée en ce que** la bande de retenue (3) s'étend jusque dans la zone de fixation (1') du support d'attache (1) et est fixée d'une part dans cette zone (1') sur la couche (9) et d'autre part sur le support d'attache (1).

6. Fermeture de couche selon l'une quelconque des revendications précédentes, **caractérisée en ce que** le dispositif de retenue (7) comprend une perforation, de préférence de la bande de retenue (3).

7. Fermeture de couche selon la revendication 6, **caractérisée en ce que** la perforation est prévue sur la face tournée vers la zone intermédiaire (1''') de la zone d'attache (1").
